# EUROPEAN PATENT APPLICATION

(11) **EP 2 277 388 A1**
(43) Date of publication of application: **26.01.2011**
(21) Application number: 09166282.5
(22) Date of filing: 23.07.2009
(51) Int. Cl.: A23L 1/00, A23L 1/03, A23L 1/30, A61K 8/97, A61K 9/14, B01J 2/02, B01J 2/04

(54) **Solid alpechin product and process for preparing a solid alpechin product**

(71) Applicant: Alsecco International, 3197 KH Rotterdam (NL)
(72) Inventor: Hofland, Gerard Willem, 2355 BG Hoogmade, (NL); Van Rosmalen, Gerda Maria, 2625 Delft, (NL)
(74) Representative: Hatzmann, Martin

(57) **Abstract**

The invention relates to a solid alpechin product comprising a powder having an average particle size in the range of from 1-500 µm, in particular 10-100 µm, as determined with scanning electron microscopy. The invention further relates to a process for preparing a solid alpechin product, comprising the subsequent steps of
- providing a feed comprising alpechin and water;
- optionally filtrating said feed to remove solids therefrom;
- optionally concentrating said feed to remove water therefrom;
- converting said feed into a spray;
- mixing said spray with supercritical carbon dioxide; and
- collecting the solid alpechin product.

## Description

The invention relates to a solid alpechin product, to the use of a solid alpechin product, to a process for preparing a solid alpechin product, to a solid alpechin product obtainable by said process.

Olive mill wastewaters (OMWW), also known as alpechines (Spanish: *alpechín*), are by-products of the olive oil industry. Alpechin usually contains 85-90 wt.% of H₂O; the remainder comprises organic compounds (in particular polyalcohols, organic acids, polysaccharides) and trace elements.

In the olive oil industry, OMWW are generally regarded to be of low commercial value and are often treated as waste. A common way to get rid of OMWW is draining them on land. However, the environment is hereby affected in a negative way. Many efforts have been undertaken to process alpechin streams as to alleviate the negative impact on the environment, not in the least because of the force of law. Another drive for processing alpechin streams is that it has been recognized that certain components of alpechines may be valuable compounds, that are suitable for application in pharmaceuticals, cosmetics and foods. An example of such application is CreAgri's Olivenol™ Polyphenol extract.

A disadvantage, however, is that the processability of alpechines is difficult. Concentrating alpechines by removing water via conventional methods usually yields syrup-like product that is difficult to handle.

Further, applying elevated temperatures for removing water may result in degradation of the alpechin components, in particular of the organic components, and so decrease the quality of the alpechin concentrate.

It is an object of the present invention to provide a novel method for processing alpechines, which does not suffer from these drawbacks.

It is in particular an object of the present invention to provide a novel method for drying alpechines, wherein lower temperatures are applied than in methods known in the art.

It is a further object to provide a processed alpechin product that comprises a lower level of degraded components, in particular a lower level of thermally degraded organic components, as compared to alpechin products known in the art.

It is in particular an object to provide a processed alpechin product, wherein the polyphenol level is high as compared to alpechin products known in the art.

It is furthermore an object to provide a processed alpechin product that is easier to handle, in particular it is an object to provide a dry and/or solid alpechin product.

It was found that one or more of these objects can be met by using a spray-drying process in which a supercritical fluid is employed.

Accordingly, the invention relates to a solid alpechin product comprising a powder having an average particle size in the range of from 1-500 µm, in particular 10-100 µm, as determined with scanning electron microscopy.
Figure 1 schematically shows an apparatus that can be used to carry out a process according to the invention.
Figure 2 schematically shows another apparatus that can carry out a process according to the invention.
Figure 3 schematically shows yet another apparatus that can carry out a process according to the invention.
Figure 4 shows by way of example how the viscosity of two different alpechin solutions depend on the shear rate.
Figure 5 shows an image of a powder according to the invention obtained with a scanning electron microscope.
Figure 6 shows another image of a powder according to the invention obtained with a scanning electron microscope.

An alpechin product according to the invention is low in water content an may be referred to a dry powder. Usually, the water content is less than 20 wt.%, preferably it is in the range of 1-18 wt.%, more preferably it is in the range of 3-17 wt.%, most preferably it is in the range of 5-15 wt.%.

A solid alpechin product according to the invention may be free-flowing. For the purpose of the invention, a powder is considered to be free-flowing when the angle of repose of the powder is less than 47°. The angle of repose of a powder is the included angle formed between the side of a cone-shaped pile of the powder and the horizontal plane.

The angle of repose may be determined by the "angle of repose test" as detailed in any standard pharmaceutical text, such as "The Theory And Practice Of Industrial Pharmacy" by Lachman, Lieberman and Kanig (Lea and Febiger, publishers).

In case a powder of alpechin product is not free-flowing, it may have an angle of repose in the range of 47° to 50°.

An alpechin product of the present invention may typically have the following composition:
protein: 5-10 wt.%;
carbohydrates: 45-68 wt.%;
organic acids: 5-10 wt.%;
fat: 17-30 wt.%;
ash 2-5 wt.%;
heavy metals: < 10 ppmw (lead: < 2 ppmw);
phenolics (polyphenols): > 6 wt.%, of which:
   hydroxytyrosol 40-50 wt.% of total phenolics
   gallic acid 2-5 wt.% of total phenolics
   tyrosol 2-5 wt.% of total phenolics
   oleuropein 5-10 wt.% of total phenolics
   other secoiridoids 10-20 wt.% of total phenolics

A solid alpechin product according to the invention may also comprise stress proteins, for example heat shock proteins.

The invention further relates to a process for preparing a solid alpechin product, comprising the subsequent steps of
- providing a feed comprising alpechin and water;
- optionally filtrating said feed to remove solids therefrom;
- optionally concentrating said feed to remove water therefrom;
- converting said feed into a spray;
- mixing said spray with supercritical carbon dioxide; and
- collecting the solid alpechin product.

The product that is obtainable by this process has the characteristics mentioned hereinabove.

It is surprising that with a method according to the invention a dry product in the form of a powder may be obtained, despite the fact that significant amounts of water are still present in the product.

Usually a precipitator is used in the process. The mixture of the spray with the supercritical carbon dioxide that is present in such precipiator usually flows from one side of the precipitator to an other side of the precipitator. In particular, the mixture flows in the direction of gravity, *i*.*e*. from the top of the precipitator to the bottom of the precipitator. The main part of the drying of the alpechin usually occurs during in the precipitator.

Usually, the mixing occurs in the precipitator, but in a specific embodiment, the mixing may also at least partly occur prior to the inlet in the precipitator. In such case, a mixture of carbon dioxide and alpechin feed may be converted into a spray.

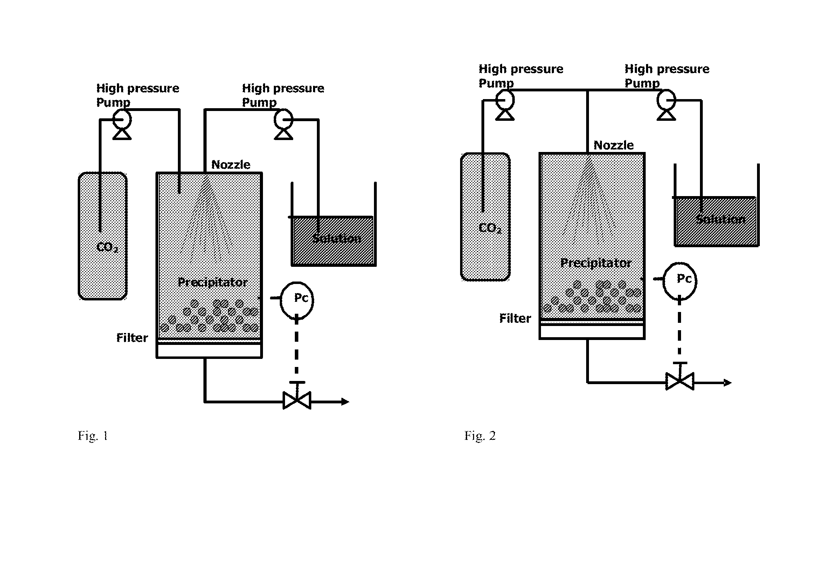

Figures 1-3 show different embodiments of an apparatus that can be used to carry out a process according to the invention, all embodiments comprising a precipitator with an inlet for a spray, a filter to collect the powder and a discharge for the exhaust carbon dioxide.
Figure 1 shows an embodiment wherein a reservoir of carbon dioxide is operatively connected to the precipitator via a high pressure pump. The precipitator comprises a separate inlet for the carbon dioxide. Further, a reservoir of alpechin is operatively connected with the precipitator via a high pressure pump. In this way, a feed comprising alpechin and water is provided, which feed may be converted into a spray by the nozzle. In this way, a spray of the feed alpechin feed may be introduced directly into the precipitator.
Figure 2 shows a variation of the embodiment of Figure 1, wherein the supply of carbon dioxide and the supply of the alpechin feed are combined before they enter the precipitator via the nozzle.

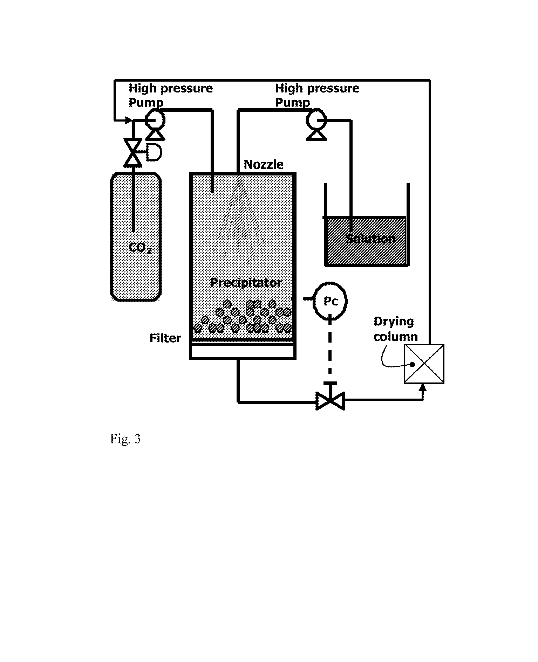

Figure 3 shows another variation of the embodiment of Figure 1,
   wherein the exhaust of carbon dioxide is operatively connected to the inlet of the carbon dioxide high pressure pump via a drying column. This drying column serves to remove the water that has been released during the drying. In this way, the carbon dioxide can be recycled after it has been used to dry the alpechin.

In a process according to the invention, the feed comprising alpechin and water usually has a water content of between 40 and 50 wt.%. The water content may be 80 wt.% or less, 60 wt.% or less, 35 wt.% or less or 20 wt.% or less. The water content may be 10 wt.% or more, 25 wt.% or more, 45 wt.% or more, or 65 wt.% or more.

Preferably, the water content of the feed comprising alpechin and water is not too low, for example not below 20 wt.% or below 10 wt%. It has been shown that it is then difficult to obtain a solid alpechin product in the form of a powder (see the example).

The feed comprising alpechin and water that is converted into a spray may have a lower water content than the raw alpechin that is obtained directly after processing olives. In a process of the invention, the alpechin feed may therefore undergo a concentration step (i.e. water removal) before it is converted into a spray, preferably a concentration step at lower temperature, e.g. a temperature of less than 50 °C or less than 40 °C. Water may be removed for example by evaporation, in particular by evaporation under reduced pressure, by reverse osmosis, by adding a drying agent (e.g. a water absorbent), or by ultrafiltation.

In a process of the invention, solid particles may be removed from the feed comprising alpechin and water prior to the conversion of the feed into a spray, in particular by microfiltation.

Usually, the water content of a solid alpechin product according to the invention depends on the temperature applied during the drying process. For example, the water content in the alpechin product may be in the range of 10-20 wt.% when temperatures between 30 °C and 50 °C have been used, and it may be in the range of 2-6 wt.% when temperatures between 70 °C and 80 °C have been used.

A process according to the invention may comprise dispersing the fluid alpechin product into another substance prior to converting it into a spray. In such way, an alpechin product may be obtained that is encapsulated in that substance. An advantage of such method is, that the obtained product may be less hygroscopic.

In a process according to the invention, the pressure during mixing is chosen such that the carbon dioxide is in a liquid (near critical) or supercritical state. Usually, the pressure is in the range of 70-200 bar. The pressure may be 75 bar or more, 100 bar or more, 125 bar or more, 150 bar or more or 175 bar or more. The pressure may be 190 bar or less, 160 bar or less, 120 bar or less or 90 bar or less.

In a process according to the invention, the temperature during mixing is chosen such that the carbon dioxide is in a supercritical state. Usually, the temperature is in the range of 25-125 °C. The temperature during the mixing is preferably 110 °C or less, more preferably 80 °C or less, and even more preferably 50 °C or less or 40 °C or less. The temperature during the mixing may be 30 °C or more, 45 °C or more, or 75 °C or more.

In addition to carbon dioxide and the alpechin feed, other components may be added to the spraying device in accordance with the present invention. For instance, compounds that reduce surface tension, such as ethanol, can be applied in such a co-spraying step to improve the yield of the process and water content of the resulting product.

The products of the invention can be used in a wide variety of applications, such as in the preparation of pharmaceutical products, such as cholesterol lowering agents and anti-cancer medicine; cosmetic products, such as creams, balms, shampoos, hair conditioners; food products or food additives, for instance in so-called neutraceuticals for providing cholesterol controlling food products.

### Examples

Two different solutions were obtained from olive processing plants in Germany and Spain, respectively. These solutions are referred to as Solution 1 and Solution 2, respectively. The water contents of each Solution 1 and 2 was determined using a Carl-Fisher coulometer and were found to be 45.1 wt.% and 45.3 wt.% respectively.

The densities of Solutions 1 and Solution 2 were determined to be 1.26 and 1.27 g/cm³, respectively.

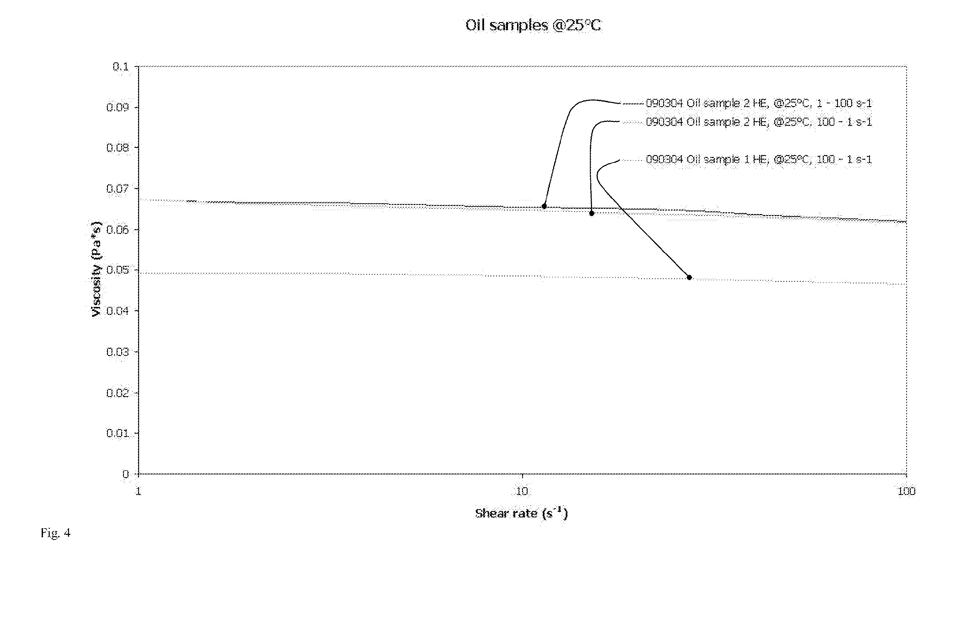

The viscosities for Solution 1 and Solution 2 were found to be 49 and 67cP (cf. olive oil which has a viscosity of approximately 80 cP). Figure 4 shows the rheologic properties of Solutions 1 and 2. Over a shear rate range of 1 to 100 s⁻¹, there was not much change in the viscosity values, although Solution 2 seemed to be slightly more non-Newtonian, showing evidence of shear-thinning. Solution 2 had more particulate material in the sample compared to Solution 1, which could explain the difference in the viscosities.

The solutions were sprayed in a set up as depicted in figure 1.

Solution 1 was sprayed into the equipment described above using flow rate of 0.5 mL/min by using a coaxial nozzle. The results evidenced that powder can be obtained after one run without further processing or drying. The production yield was around 65 wt.% based on dry product.

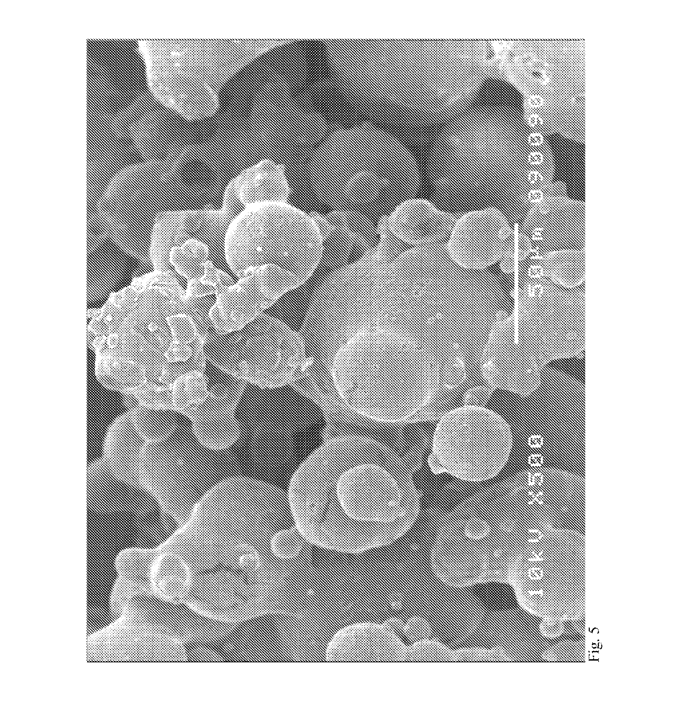

SEM analysis of the dried powder showed agglomerated particles ranging in size from 10 to 100µm. The SEM image is shown in figure 5. The water content of the final powder was also determined by means of Denver apparatus. There were 3 different drying steps investigated: 60°C/70°C; 70°C/80°C and 70°C/110°C. Up to 80°C, only 4% water was detected.

One sample of Solution 1 was further concentrated (to 10 wt.% H₂O, the water was removed by film evaporation) and the concentrated sample was processed under the same operating conditions as mentioned above. The resultant material was not sticky and could not obtained in the form of a powder. It absorbed water quickly from the atmosphere. Injection of the feed was also rather difficult due to the higher viscosity of the solution stream.

Solution 2 was subjected to the same operating conditions as for Solution 1 described above. The resultant product of Solution 2 was a powder material but slightly more sticky than Solution 1. After drying in a step-wise method - 70°C/110°C - the resultant water content was 5.6%

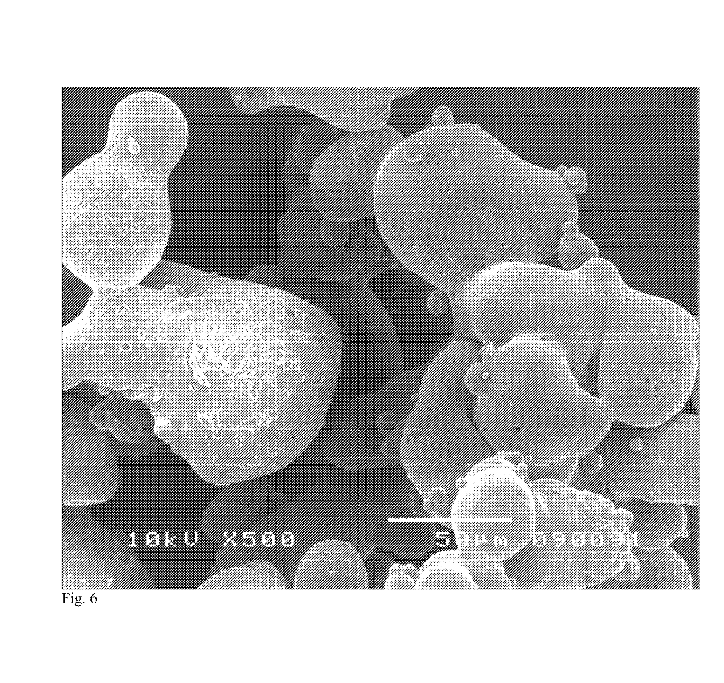

The SEM image of this sample is given in figure 6. It can be observed that the structure of the powder product for Solution 2 is very similar to that for Solution 1.

Both powders obtained from processing Solution 1 or 2 were very hydroscopic and required fast harvesting from the filter and storage in well-closed bottles. Upon leaving the material on the filter in direct contact with air, the powder becomes wet within several minutes.

Further tests involved introduction of ethanol into the system to assist with the drying process (co-spraying). By filling the spraying vessel with ethanol, the resultant product from Solution 2 was a dry powder, which was easy to harvest.

## Claims

1. Solid alpechin product comprising a powder having an average particle size in the range of from 1-500 µm, in particular 10-100 µm, as determined with scanning electron microscopy.

2. Product according to claim 1, having a water content of less than 20 wt.%, preferably of 5-15 wt.%.

3. Product according to any one of the previous claims, wherein the powder is a free-flowing powder.

4. Product according to any one of the previous claims, having the following composition: proteins: 5-10 wt.%; carbohydrates: 45-68 wt.%; organic acids: 5-10 wt.%; fat: 17-30 wt.%; ash 2-5 wt.%; heavy metals: < 10 ppmw (of which lead: < 2 ppmw); and phenolics (polyphenols): > 6 wt.%, of which:
hydroxytyrosol 40-50 wt.% of total phenolics, gallic acid 2-5 wt.% of total phenolics, tyrosol 2-5 wt.% of total phenolics, oleuropein 5-10 wt.% of total phenolics, and other secoiridoids 10-20 wt.% of total phenolics.

5. Use of a solid alpechin product according to any one of the previous claims in the preparation of a pharmaceutical product, a cosmetic product, a food product or a food additive.

6. Process for preparing a solid alpechin product, comprising the subsequent steps of
- providing a aqueous feed comprising alpechin;
- optionally filtrating said feed to remove solids therefrom;
- optionally concentrating said feed to remove water therefrom;
- converting said feed into a spray;
- mixing said spray with supercritical carbon dioxide; and
- collecting the solid alpechin product.

7. Process according to the previous claim wherein said filtration step is carried out using microfiltration and/or ultrafiltration; and/or wherein said concentration step is carried out using evaporation.

8. Process according to any one of the claims 6-7, wherein a mixture comprising carbon dioxide and water is obtained, the process further comprising
- removing the water from said mixture; and
- using the carbon dioxide for mixing the spray with supercritical carbon dioxide.

9. Process according to any one of the claims 6-8, wherein the temperature during the mixing is in the range of 30-50 °C.

10. Process according to any one of the claims 6-9, wherein the pressure during mixing is in the range of 70-200 bar.

11. Process according to any one of the claims 6-10, which process is a continuous process.

12. Process according to any one of the claims 6-11, wherein, during and/or after the collecting, the solid alpechin product is dosed and packaged under an atmosphere, comprising a gas selected from the group of nitrogen and carbon dioxide.

13. A solid alpechin product obtainable by a process according to any of the claims 6-12.
